# EUROPEAN PATENT SPECIFICATION

(11) **EP 0 614 355 B1**
(45) Date of publication and mention of the grant of the patent: **30.01.2002**
(21) Application number: 93900706.8
(22) Date of filing: 25.11.1992
(51) Int. Cl.: A61K 9/127, A61K 39/00

(54) **ANTITUMOR VACCINES**
ANTITUMORVAKZINE
VACCINS ANTITUMORAUX

(30) Priority: 26.11.1991 US 800474
(43) Date of publication of application: 14.09.1994
(73) Proprietor: JENNER TECHNOLOGIES, Tiburon, CA 94920 (US)
(72) Inventor: SPITLER, Lynn, E., Tiburon, CA 94920 (US)
(74) Representative: Sexton, Jane Helen
(86) International application number: PCT/US92/10264
(87) International publication number: WO 93/10763

(56) References cited:
- US-A- 4 343 895
- US-A- 4 557 931
- US-A- 5 026 557
- DATABASE WPI Week 9035 Derwent Publications Ltd., London, GB; AN 90-266194 XP002036805 & JP 02 188 532 A (OTSUKA PHARMA CO LTD) , 24 July 1990
- J. GEN. VIROL., vol. 65, 1984, LONDON (GB), pages 1009-1014, XP002036804 A.R. NEURATH ET AL.: "antibodies to hepatitis B surface antigen (HBsAg) elicited by immunization with a synthetic peptide covalently linked to liposomes"
- Hybridoma, Vol. 8, No. 4, 1989, (SELA), "Colon Carcinoma-Associated Glycoproteins Recognized by Monoclonal Antibodies Co-029 and GA22-2", pages 481-491, see the Abstract.
- Proc. Natl. Acad. Sci. USA, Vol. 87, pages 3542-3546, May 1990, (SZALA), "Molecular Cloning of CDNA for the Carcinoma-Associated Antigen GA733-2", see the Abstract.
- Methods in Enzymology, Vol. 149, 1987, (HEATH), "Covalent Attachment of Proteins to Liposomes", pages 111-119, see page 111.

## Description

### Field of the Invention

The invention relates to the field of cancer therapeutic protocols and the treatment and prevention of cancer. More specifically, the invention concerns antitumor vaccines comprising a GA733-2 antigen which is encapsulated in, or coupled to, liposomes.

### Background Art

cancer accounts for almost 500,000 deaths each year in the United States, and more than 1,000,000 new cases of cancer are diagnosed annually. As the average lifespan increases, the incidence of cancer increases as well. Despite the magnitude of the problem, treatment strategies have been slow to evolve, and are most typically invasive, painful, and associated with long-term severe discomfort. Surgery, chemotherapy and radiation have met with limited success while producing severe side effects and dramatic lowering of quality of life. There is clearly a need for more effective treatment protocols.

The use of tumor-associated antigens derived from the subject's own tumor, formulated in a liposome composition, and applied in treatment protocols has been disclosed in Japanese patent application JP 2,188,532. This application describes extraction and partial purification of tumor-associated antigens and their reconstitution into liposome compositions, as well as the use of these compositions in tumor treatment. The use of extracted tumor-associated antigens as a vaccine when associated with liposomes has also been described by Phillips, N.C., et al., Cancer Det & Prev (1990) 14:491-496. The use of liposomes as adjuvants for vaccines generally is described in U.S. Patent Nos. 4,891,208 and 4,721,612, and in European application 036,277. Tumor-associated antigens, also extracted from tumors, have been encapsulated in liposomes for the purpose of *ex vivo* testing, as described in U.S. Patent 4,343,895. Covalent attachment of proteins to liposomes in general is described by Heath, T.D., Meth Enzymol (1987) 149:111-119.

Tumor-associated antigens which characterize tumors of a particular histological type have also been described. Sela, B.-A., et al., Hybridoma (1989) 8:481-491, describe antibodies and precipitated antigens for the gastrointestinal tumor antigen CO-029 and GA22-2. Szala, S., et al., describe the cloning of a cDNA for a carcinoma-associated antigen, GA733-2. In general, antigens broadly associated with a tumor type characterized by a particular histology have been identified and purified. The GA733-2 antigen is useful in the vaccines of the invention.

US patent 4,557,931 discloses GM2 which is a ganglioside present on the surface of tumors and which stimulates an appreciable immune response in mammals. It is said to be useful when coupled with a non-toxic protein carrier or mixed with an adjuvant and injected parenterally in raising the anti-GM2 titer in serum.

US patent 5,026,557 discloses an adjuvant composition comprising a small liposome with incubation associated immunopotentiator such as lipid A and an incubation associated antigen such as a ganglioside antigen.

### Disclosure of the Invention

The invention provides vaccine compositions which are useful in the prevention and treatment of tumors that express the GA733-2 antigen. The compositions employ, as active ingredient, a GA733-2 antigen. The antigen is prepared independently of the tumor itself. Thus, the antigen may be prepared recombinantly in heterologous hosts, may be generated *in situ* from a viral vector, or may be prepared in the form of a mimicking antiidiotypic antibody using the antigen as an immunogen in an initial immunization protocol. The independently prepared antigen is then encapsulated in or covalently coupled to liposomes to enhance its effectiveness.

Accordingly, in one aspect, the invention is directed to an antitumor vaccine composition comprising, as active ingredient, a GA733-2 antigen. The composition contains the active ingredient encapsulated in, or coupled to, liposomes.

In other aspects, the invention is directed to the use of the GA733-2 in the manufacture of a medicament comprising said GA733-2 antigen encapsulated in, or covalently bound to, liposome carrier for vaccinating against a tumor that expresses the GA733-2 antigen.

The antitumor vaccine compositions of the invention have two essential components: a GA733-2 antigen and a liposome preparation. Adjuvants and/or cytokines, as well as other excipients may also be included. The vaccine composition may further contain an additional synthetically prepared tumor-associated antigen. While, for practical reasons, the tumor-associated antigen is typically an antigen common to tumor in many subjects wherein the tumor is characterized by a given histological type, there is no theoretical reason why antigens associated uniquely with a tumor in a given subject could not be independently prepared and used in the vaccine as well. However, preparation of such individual antigens independent of the tumor *per se is*, at present, impractical, since such preparation would require purification and characterization of the unique antigen for each individual tumor. The vaccines of the invention, therefore, employ antigens common to tumors in subjects which share tumor types of a given histology. Representative of such antigens are CO-029, which is associated with tumors of the gastrointestinal tract, colorectum and pancreas, and GA 733-2, which is associated with tumors of the gastrointestinal tract, prostate, cervix, ovary, bladder, lung, breast, colorectum and pancreas. Such common antigens can readily be prepared independent of the tumor using synthetic methods, e.g., recombinantly or by solid-phase peptide synthesis. Other "synthetic" methods of preparation include generation of antibodies whereby the antigen is mimicked by antiidiotypic monoclonal antibodies. The antigens may also be generated *in situ* employing encoding DNA inserted into viral vectors.

By "synthetically prepared" is meant generation of the antigen by means independent of the tumor which it characterizes or from which it might be extracted -- i.e., the antigen is not isolated from its histologically marked tumor or other tumor.

### Preparation of the Liposome Component

The preparation of liposomes per se is well known in the art; a variety of techniques are available for the preparation of liposomes from phospholipids or other lipid substances and mixtures thereof. Liposomes are commonly characterized as unilamellar or multilamellar. The use of such liposomes is helpful in targeting the active therapeutic agent to the reticuloendothelial system, one of the sites of generation of immune response in mammalian hosts.

For formation of the liposomes, any lipid capable of forming vesicles can be employed. For clinical application, it is desirable that the lipid be nontoxic, physiologically acceptable, and metabolizable. Common bilayer-forming lipids having clinical potential are phospholipids, fatty acids, sphingolipids, glycosphingolipids, and steroids, especially glycerol-containing phospholipids. Especially preferred are phosphatidylcholine or lecithin, or cholesterol and its derivatives.

The tendency of liposomes to aggregate and fuse can be controlled by the inclusion of small amounts of acidic or basic lipids in the formulation. The characteristics of liposomes are determined by the properties of the components, such as hydrocarbon chain length, degree of unsaturation of the hydrocarbon chain, degree of branching of the hydrocarbon chain and the presence of ionic moieties. The temperature of the system is also relevant.

Multilamellar liposomes can be created by depositing a mixture of lipids as a thin film by evaporation under reduced pressure followed by dispersion with an excess volume of aqueous buffer with or without organic solvents. Another method is to mix the aqueous phase with small unilamellar liposomes followed by lyophilization. The multilamellar liposomes are formed when the lyophilized product is rehydrated, usually with a small amount of distilled water. The small unilamellar liposomes to be used in this process are produced by dispersing the lipids in an aqueous medium followed by a mechanical means of dispersion such as sonication, use of a high pressure device, or a solvent injection method. Large and intermediate-sized unilamellar liposomes can also be produced by conventional techniques including detergent dialysis, extrusion through small pore size membranes under high pressure, freeze thawing followed by slow swelling, dehydration followed by rehydration and dilution; or dialysis of lipids in the presence of chaotropic ions. The size of the liposomes can be made more uniform by fractionation procedures such as centrifugation or size-exclusion chromatography, homogenization, or capillary pore membrane extrusion.

The target within the host and major site of uptake of liposomes is the reticuloendothelial system, mainly in the liver, spleen, and lung of the host. Factors affecting uptake of liposomes by the host include liposome size, stability and surface charge. The apparent optimal size for targeting the reticuloendothelial system is >1.0 µm, particularly for targeting liver, lung, and spleen.

### Preparation of the Tumor-Associated Antigens

A number of tumor-associated antigens common to tumors in various individuals have been isolated and purified and the relevant cDNA cloned. It is expected that additional such antigens will be purified in the future and the DNA obtained. Once the structure of the antigen is identified, standard solid-phase techniques may be used to prepare protein antigens, as is well understood in the art.

Alternatively, the antigens may be prepared using recombinant techniques from the relevant cDNA. General techniques for recombinant expression in a variety of host systems such as mammalian cells, insect cells, yeast cells, bacterial cells and even plant cells are by now well known in the art. For example, a baculovirus/insect cell system is described in U.S. Patent 4,879,236.

In addition to recombinant production and recovery of the protein antigen and subsequent formulation into a vaccine, the liposome components of the preparation can be used to encapsulate a viral production system for the antigen whereby the antigen will be produced *in situ*. For this approach the DNA encoding the antigen is inserted into operable linkage with expression regulators in a viral vector and the recombinant vector is formulated into the vaccine for administration. The antigen is then generated in the subject by expression from the viral vector *in situ*.

Methods for utilization of viral vectors in vaccines for *in situ* generation of antigens is well known. For example, such techniques are described for Vaccinia virus by Hruby, D.E., Vet Parasitol (1988) 29:281-292; and use of fowl pox virus as a host vector is described by Taylor, J. et al., Vaccine (1991) 9:190-193.

The use of viral vectors in AIDS vaccines was also described by Iiu, S.-L., in "AIDS Research Reviews", Vol. 1 (1991), Marcel Dekker Inc., New York, publishers, pages 403-416.

Finally, a tumor-independent source of the tumor-associated antigen is found in the form of monoclonal antiidiotypic antibodies which mimic the contours of the antigen. Production of antiidiotypic antibodies is described, for example, by Goding, J.W., in "Monoclonal Antibodies, Principles and Practice", 2d Ed., Academic Press (1986). While generally these antibodies are initially produced in nonhuman hosts, the constant regions of these antibodies may be "humanized" using chimeric recombinant techniques. Such antiidiotypic monoclonal antibodies to tumor antigens have been produced, for example, by Austin et al., Immunology (1989) 67:525-530.

A number of tumor-associated antigens have been purified and characterized, including CO-029 and GA 733-2, as described above, as well as antigens characterizing melanoma, as described by Kahn et al., Cancer Res (1989) 49:3157-3162; and by Kusama et al., J Immunol (1989) 143:3844-3852. Carcinoembryonic antigen (CEA) is described by Vialo *et* al., J Immunol (1987) 139:4250. Any tumor-associated antigen which can be isolated and characterized to permit its production independent of its tumor cell source can be used.

### Preparation of the Vaccines and Administration

The antitumor vaccine compositions are prepared by encapsulating a GA733-2 antigen and optionally an additional tumor-associated antigen into the liposomes or by covalently bonding them thereto. For encapsulation, intermediate-sized liposomes are preferred. Either water-soluble or lipophilic antigens are added to the aqueous component before formation of the liposomes in order to entrap the antigen either into the encapsulated aqueous space within the liposomes or to entrap the antigen in the lipid bilayer, as the case may be. Methods to couple antigens covalently to liposomes are also well known in the art, and are disclosed, for example, by Torchilin et al., Biochem Biophys Res Comm (1978) 85:983. Preferred methods employ aqueous media. For example, carboxyl groups activated with N-hydroxysuccinimide may be reacted with amino groups to produce amides. Pyridyldithiols are reacted with thiols to produce disulfide bonds, or maleimide derivatives are reacted with thiols to produce thioethers. In the alternative, the antigens may be coupled to individual lipid molecules and then inserted into preformed liposomes.

The resulting liposomal formulations include GA733-2 antigen and may also include one or several other synthetically produced antigens. They may also be supplemented with adjuvants such as LPS, lipid A, muramyldipeptide, and the like, as described in "Liposomes", Ostro, M.J., ed., Marcel Dekker Inc. (1983), page 249; or with polysaccharides. The formulations may also be formulated with immune-enhancing cytokines such as IL-1, IL-2, IL-12, GM-CSF, G-CSF and γ-interferon.

The resulting liposomal formulations are administered using conventional methods, commonly by injection or by transmucosal administration. Injection may be by conventional routes, including intravenous, intraperitoneal, subcutaneous, and intramuscular; intravenous injection is preferred. Aerosol administration may also be convenient, provided agents are added to the composition to effect transmucosal transport, such as fusidic acid, bile salts, detergents, and the like. The antigens are administered in doses ranging from 0.01 µg to 100 mg, preferably 0.1 µg to 10 mg, and more preferably 10 µg to 1 mg per dose in a volume of 0.1 to 5 ml for parenteral administration. Multiple doses may be administered as frequently as once a week for the first year with booster inoculations occurring every six months to five years thereafter.

The following examples are intended to illustrate the invention.

### EXAMPLE I

Disterolphosphatidylcholine, cholesterol, and dicetyl phosphate and methanol are used in a volumetric ratio of 5:1 in lipid solvents. Multilamellar liposomes (MLV) are prepared by the method described by Bangham, et al., with some modifications. Disterolphosphatidylcholine (70 mol%), cholesterol (24 mol%), and dicetyl phosphate (6 mol%) are mixed in a round bottomed flask and the lipid solvents are evaporated to dryness under a stream of nitrogen. The aqueous phase, containing GA733-2 in PBS, is added to the thin film of dried lipids in a flask at 42° C. (Lipophilic proteins may be subjected to detergent dialysis techniques well known to those skilled in the art as part of the encapsulation technique.) After 30 minutes of swelling time, the dispersion is completed at 42° C by adding a few glass beads and vortexing the mixture vigorously for 15 minutes. The MLV liposomes thus formed are equilibrated for 2 hours at room temperature, purified, and used immediately.

The MLV liposomes are sized by a laser light sorter. Liposome preparations are passed through a Sephadex G-75 column to separate liposome-incorporated antigens from free proteins. Liposomes are eluted with void volume followed by unencapsulated protein. To estimate the efficacy of protein encapsulation in the vesicles, membrane antigens are labeled with ¹²⁵I iodobeads. Free ¹²⁵I is removed by extensive dialysis. The ¹²⁵I-labeled proteins are used in the aqueous phase of MLV preparation described above and are passed through a Sephadex G-75 column. The percentage of radioactivity eluting with liposomes is taken as an index of encapsulation efficiency. Liposome compositions for use in mammalian hosts are prepared without radiolabeling. Under these conditions, the protein concentration is determined by protein assay as known to those of skill in the art. The GA733-2-liposome encapsulated compositions are stored at 4° C under argon until formulation for administration into a mammalian host.

### EXAMPLE II

Liposomes are prepared with phosphatidylcholine, cholesterol, and dicetylphosphate used in a molar ratio of 7:2:1. The lipids in chloroform:methanol (95:5) are brought to dryness in a round bottom flask by rotary evaporation under argon. The dried film of lipids are resuspended in 2ml of 0.15M borate saline buffer at pH 8 containing 150 mg of GA733-2 TAA trace labeled with ¹²⁵I. The suspension is allowed to incubate for 1 hour, sonicated in a bath type sonicator for 1 hour, and then left for a third hour before being loaded onto a lipid presaturated Sephadex G-200 column to remove the non-liposome-associated protein. The TAA content of the liposome peak is determined by the specific activity of the ¹²⁵I protein. Liposome compositions for use in mammalian hosts are prepared without radiolabeling and the protein concentration determined as previously described. The GA733-2-liposmoe encapsulated compositions are stored at 4° C under argon until formulation for administration into a mammalian host.

### EXAMPLE III

One mg of GA733-2, 75 mg of POPC (1-palmitoyl-2oleoyl-phosphatidylcholoine), and 175 ng of DOPG (1,2-dioleoyl phosphatidyl glycerol) in a total volume of 2.5 ml of tert-butanol are aliquoted into a 5 ml non-pyogenic vials, frozen at 4° C, and lyophilized over a period of 16 hours. The final preparations are stored at 4°C under argon and remain stable for several months. For injection, the liposomes are reconstituted in 2.5 ml of sterile saline, added to the vial and allowed to sit for 15 seconds at room temperature. The vial is then vortexed for 30 seconds. This procedure results in liposomes in the size range of 0.1 to 3 µm.
^{*} Not of the invention.

### EXAMPLE IV^{*}

Disterolphosphatidylcholine, cholesterol, and dicetyl phosphate and methanol are used in a volumetric ratio of 5:1 in lipid solvents. Multilamellar liposomes (MLV) are prepared by the method described by Bangham, et al., with some modifications. Disterolphosphatidylcholine (70 mol%), cholesterol (24 mol%), and dicetyl phosphate (6 mol%) are mixed in a round bottomed flask and the lipid solvents are evaporated to dryness under a stream of nitrogen. The aqueous phase, containing CO-029 in PBS, is added to the thin film of dried lipids in a flask at 42° C. (Lipophilic proteins may be subjected to detergent dialysis techniques well known to those skilled in the art as part of the encapsulation technique.) After 30 minutes of swelling time, the dispersion is completed at 42° C by adding a few glass beads and vortexing the mixture vigorously for 15 minutes. The MLV liposomes thus formed are equilibrated for 2 hours at room temperature, purified, and used immediately.

The MLV liposomes are sized by a laser light sorter. Liposome preparations are passed through a Sephadex G-75 column to separate liposome-incorporated antigens from free proteins. Liposomes are eluted with void volume followed by unencapsulated protein. To estimate the efficacy of protein encapsulation in the vesicles, membrane antigens are labeled with ¹²⁵I iodobeads. Free ¹²⁵I is removed by extensive dialysis. The ¹²⁵I-labeled proteins are used in the aqueous phase of MLV preparation described above and are passed through a Sephadex G-75 column. The percentage of radioactivity eluting with liposomes is taken as an index of encapsulation efficiency. Liposome compositions for use in mammalian hosts are prepared without radiolabeling. Under these conditions, the protein concentration is determined by protein assay as known to those of skill in the art. The CO-029-liposome encapsulated compositions are stored at 4° C under argon until formulation for administration into a mammalian host.
^{*} Not of the invention.

### EXAMPLE V^{*}

Liposomes are prepared with phosphatidylcholine, cholesterol, and dicetylphosphate used in a molar ratio of 7:2:1. The lipids in chloroform:methanol (95:5) are brought to dryness in a round bottom flask by rotary evaporation under argon. The dried film of lipids are resuspended in 2ml of 0.15M borate saline buffer at pH 8 containing 150 mg of CO-029 TAA trace labeled with ¹²⁵I. The suspension is allowed to incubate for 1 hour, sonicated in a bath type sonicator for 1 hour, and then left for a third hour before being loaded onto a lipid presaturated Sephadex G-200 column to remove the non-liposome-associated protein. The TAA content of the liposome peak is determined by the specific activity of the ¹²⁵I protein. Liposome compositions for use in mammalian hosts are prepared without radiolabeling and the protein concentration determined as previously described. The CO-029-liposmoe encapsulated compositions are stored at 4° C under argon until formulation for administration into a mammalian host.

Liposomes are prepared with phosphatidylcholine, cholesterol, and dicetylphosphate used in a_molar ratio of 7:2:1. The lipids in chloroform:methanol (95:5) are brought to dryness in a round bottom flask by rotary evaporation under argon. The dried film of lipids are resuspended in 2ml of 0.15M borate saline buffer at pH 8 containing 150 mg of CO-029 TAA trace labeled with ¹²⁵I. The suspension is allowed to incubate for 1 hour, sonicated in a bath type sonicator for 1 hour, and then left for a third hour before being loaded onto a lipid presaturated Sephadex G-200 column to remove the non-liposome-associated protein. The TAA content of the liposome peak is determined by the specific activity of the ¹²⁵I protein. Liposome compositions for use in mammalian hosts are prepared without radiolabeling and the protein concentration determined as previously described. The CO-029-liposmoe encapsulated compositions are stored at 4° C under argon until formulation for administration into a mammalian host.
^{*} Not of the invention.

### EXAMPLE VI^{*}

One mg of CO-029, 75 mg of POPC (1-palmitoyl-2oleoyl-phosphatidylcholoine), and 175 ng of DOPG (1,2-dioleoyl phosphatidyl glycerol) in a total volume of 2.5 ml of tert-butanol are aliquoted into a 5 ml non-pyogenic vials, frozen at 4° C, and lyophilized over a period of 16 hours. The final preparations are stored at 4° C under argon and remain stable for several months. For injection, the liposomes are reconstituted in 2.5 ml of sterile saline, added to the vial and allowed to sit for 15 seconds at room temperature. The vial is then vortexed for 30 seconds. This procedure results in liposomes in the size range of 0.1 to 3 µm.

### EXAMPLE VII

The TAA GA-733-2 is conjugated to the surface of liposomes according to techniques described by Heath, T.D., *supra.* Essentially, saturated synthetic phosphatidylethanolamine conjugation lipid is activated by drying down 100 micromole of the lipid in a suitable reaction vessel. Lipid is redissolved in 10 ml of dry chloroform. 300 micromoles of triethylamine is added followed by 150 micromole of N-succinimidylpyridyldithiopropionate in 5 ml methanol. The mixture is stirred at room temperature under argon. The progress of the reaction is checked by thin layer chromatography using silica gel plates run in chloroform (65):methanol (25):water(4). The derivative gives a faster running spot than phosphatidylethanolamine and the spots are visualized with a phosphomolybdate spray. The reaction is complete when no more phosphatidylethanolamine is detected on the plates, generally 1 to 2 hours after initiation. The mixture is dried down, resuspended in chloroform, and applied to a 10 gram silicic acid column equilibrated with chloroform. The column is washed with 20 ml of chloroform and eluted with 20 ml portions of chloroform:methanol, first at 95:5, then at 90:10, then at 85:15, and finally at 80:20. Five ml fractions are collected and the pure derivative located by thin layer chromatography. The fractions are pooled, concentrated by evaporation at reduced pressure in a rotary evaporator, and rechecked for purity *by* thin layer chromatography. The product is stored at -20° C under argon in chloroform solution in sealed ampules.

. GA-733-2 is thiolated with N-succinimidylpyridyldithiopropionate. A solution of aggregate-free GA-733-2 is prepared in 0.1 M sodium phosphate and 0.1 M sodium chloride, at pH 7.5. The GA-733-2 concentration is about 2-6 mg/ml. N-succinimidylpyridyldithiopropionate solution is prepared in ethanol at 20 micromole/ml. N-succinimidylpyridyldithiopropionate is then added dropwise to the GA-733-2 solution to give a molar ratio of 15:1 N-succinimidylpyridyldithiopropionate:GA-733-2, wherein the ethanol concentration does not exceed 1%. The mixture is allowed to react at room temperature for 30 minutes. The product is separated from the reactants by gel chromatography on Sephadex G-50 equilibrated with 0.05 M sodium citrate, 0.05 M sodium phosphate, 0.05 M sodium chloride, pH 7.0. The thiolated GA-733-2 is characterized by measuring the number of pyridylthiols per molecule as described by Carlsson et al., Biochem. J. (1978)173:723. The pyridyldithio-GA-733-2 product is stored at 4° C following sterilization by filtration.

Just prior to conjugation, the pyridyldithio-GA-733-2 is reduced to generate thiols by placing the product in citrate phosphate buffer pH 7.0 and titrating to pH 4.5 by the addition of small amounts of 1N HCL A 2.5 M solution of dithiothreitol is prepared in 0.1 M acetate buffer pH 4.5, and 10 µl of this solution are added to each ml of the protein solution. After 30 minutes the protein solution is separated from the dithiothreitol by gel chromatography on a Sephadex G-75 column equilibrated with pH 6.7 buffer purged with nitrogen or argon to remove dissolved oxygen. The protein fractions are collected under inert gas to exclude oxygen.

Liposomes are prepared by any known technique, for example from phosphatidylcholine and cholesterol in 1:1 or 2:1 molar ratio. The conjugation lipid is added to the other lipids at a concentration of 1-5 mole per 100 mole of lipid.

Thiolated GA-733-2 is conjugated to the liposomes by mixing at pH 6.0 - 8.0 and the reaction allowed to proceed overnight. Unreacted thiols are blocked with Ellman's reagent and liposomes separated from non-conjugated protein by gel chromatography or centrifugation, preferably by centrifugation to avoid loss of liposomes on the gel matrices. The conjugate compositions are stored at 4° C under until formulation for administration into a mammalian host.
*Not of the invention.

### EXAMPLE VIII^{*}

The TAA CO-029 is conjugated to the surface of liposomes according to techniques described by Heath, T.D., *supra*. Essentially, saturated synthetic phosphatidylethanolamine conjugation lipid is activated by drying down 100 micromole of the lipid in a suitable reaction vessel. Lipid is redissolved in 10 ml of dry chloroform. 300 micromoles of triethylamine is added followed by 150 micromole of N-succinimidylpyridyldithiopropionate in 5 ml methanol. The mixture is stirred at room temperature under argon. The progress of the reaction is checked by thin layer chromatography using silica gel plates run in chloroform(65):methanol(25):water(4). The derivative gives a faster running spot than phosphatidylethanolamine and the spots are visualized with a phosphomolybdate spray. The reaction is complete when no more phosphatidylethanolamine is detected on the plates, generally 1 to 2 hours after initiation. The mixture is dried down, resuspended in chloroform, and applied to a 10 gram silicic acid column equilibrated with chloroform. The column is washed with 20 ml of chloroform and eluted with 20 ml portions of chloroform:methanol, first at 95:5, then at 90:10, then at 85:15, and finally at 80:20. Five ml fractions are collected and the pure derivative located by thin layer chromatography. The fractions are pooled, concentrated by evaporation at reduced pressure in a rotary evaporator, and rechecked for purity by thin layer chromatography. The product is stored at -20° C under argon in chloroform solution in sealed ampules.

CO-029 is thiolated with N-succinimidylpyridyldithiopropionate. A solution of aggregate-free CO-029 is prepard in 0.1 M sodium phosphate and 0.1 M sodium chloride, at pH 7.5. The CO-029 concentration is about 2-6 mg/ml. N-succinimidylpyridyldithiopropionate solution is prepared in ethanol at 20 micromole/ml. N-succinimidylpyridyldithiopropionate is then added dropwise to the CO-029 solution to give a molar ratio of 15:1 N-succinimidylpyridyldithiopropionate:CO-029, wherein the ethanol concentration does not exceed 1%. The mixture is allowed to react at room temperature for 30 minutes. The product is separated from the reactants by gel chromatography on Sephadex G-50 equilibrated with 0.05 M sodium citrate, 0.05 M sodium phosphate, 0.05 M sodium chloride, pH 7.0. The thiolated CO-029 is characterized by measuring the number of pyridylthiols per molecule as described by Carlsson et al., Biochem. J. (1978)173:723. The pyridyldithio-CO-029 product is stored at 4° C following sterilization by filtration.

Just prior to conjugation, the pyridyldithio-CO-029 is reduced to generate thiols by placing the product in citrate phosphate buffer pH 7.0 and titrating to pH 4.5 by the addition of small amounts of 1N HCl. A 2.5 M solution of dithiothreitol is prepared in 0.1 M acetate buffer pH 4.5, and 10 µl of this solution are added to each ml of the protein solution. After 30 minutes the protein solution is separated from the dithiothreitol by gel chromatography on a Sephadex G-75 column equilibrated with pH 6.7 buffer purged with nitrogen or argon to remove dissolved oxygen. The protein fractions are collected under inert gas to exclude oxygen.

Liposomes are prepared by any known technique, for example from phosphatidylcholine and cholesterol in 1:1 or 2:1 molar ratio. The conjugation lipid is added to the other lipids at a concentration of 1-5 mole per 100 mole of lipid.

Thiolated CO-029 is conjugated to the liposomes by mixing at pH 6.0 - 8.0 and the reaction allowed to proceed overnight. Unreacted thiols are blocked with Eliman's reagent and liposomes separated from non-conjugated protein by gel chromatography or centrifugation, preferably by centrifugation to avoid loss of liposomes on the gel matrices. The conjugate compositions are stored at 4° C under until formulation for administration into a mammalian host.

### EXAMPLE IX

Anti-idiotypic or Ab2 monoclonal antibodies (MAb) mimicking either GA733-2 or CO-029 tumor associated antigens are produced by techniques known to those skilled in the art. (See, e.g. Goding, J.W., Monoclonal Antibodies: Principles and Practice, 2 ed., Academic Press (1986)). Essentially, either protein, or the tumor cell line from which the protein is derived, is employed as an immunogen to produce anti-antigen monoclonal antibody or Ab1 in a mammalian host, usually murine, and, most frequently, Balb C mice. The protein or cell line may be injected with or without an adjuvant such as complete or incomplete Freund's adjuvant or the like. Monoclonal antibody(Ab1) is then employed as an immunogen to produce Ab2 anti-idiotypic monoclonal antibodies by similar methods. Alternatively, existing MAb to either antigen may be employed as immunogen to produce Ab2 such as MAb GA733 reported by Herlyn, et al., Science (1986)232:100-02.

Anti-TAA Ab2 antibodies are encapsulated into liposomes by techniques well known to those skilled in the art. Alternatively, such immunoglobulin molecules may be conjugated to the surface of liposomes as described above.

### EXAMPLE X

Normal mice display the murine analog of GA733-2 and CO-029 (>80% homology) in a tissue distribution similar to the distribution of the antigen in humans. In addition, a murine tumor, e.g. P815 or CT3, is transfected by techniques well known to those skilled in the art with the cDNA encoding the human GA733-2 and CO-029 antigens resulting in the stable expression of the human gene products on the surface of the murine tumors. The tumors are subsequently introduced into normal mice and proliferate while continuing to express the recombinant antigens. This model allows testing of both safety and efficacy of the subject liposomal vaccines.

For safety testing, either normal or tumor bearing mice are vaccinated with various formulations of either GA733-2, CO-029 or anti-idiotypic monoclonal antibodies in any of the subject liposomal compositions in doses ranging from 0.01µg to 100mg, usually 0.1µg to 10mg, more usually 10µg to 1mg total antigen per dose up to six doses. Vaccination is administered i.p., i.m., s.c., or via foot pad in volumes of 10µl to 2ml. Results are assessed by serial determinations of weight, clinical observations, laboratory parameters including complete blood counts, chemistries, and gross histopathology at necropsy.

For efficacy testing, mice are subjected to the same immunization regimen prior to, during, or post tumor implantation. Efficacy is measured by 1) determinations of tumor growth following subcutaneous implantations, 2) enumeration of tumor nodules in a micro-metastatic model and/or 3) gross survival. Efficacy is also measured by determination of immunological responses including but not limited to antigen specific antibody titer, delayed type hypersensitivity, ADCC, and cytotoxic T lymphocytes.

### EXAMPLE XI

Patients with cancer may have the cancer surgically excised and then be given the subject tumor vaccines. If clinical, laboratory, and radiologic examination reveals no evidence of gross disease, the treatment is considered to be "surgical adjuvant treatment." Vaccinations can also be commenced prior to surgery, in which case it is considered 'neoadjuvant therapy." Patients with cancer who develop metastatic disease which is not surgically resected can also be treated.

Patients are vaccinated with various formulations of either GA733-2, CO-029, or anti-idiotypic monoclonal antibodies in any of the subject compositions. The route may be intramuscular (i.m.), subcutaneous (s.q.), or intradermal (i.d.). The dose of antigen administered is 0.01 µg to 100mg, usually 0.1µg to 10mg, more usually 10µg to 1mg per dose in a volume of 0.1 to 5 ml for parenteral administration. The vaccine is administered as often as weekly for the first 6 months and as infrequently as monthly, bimonthly, or every 3 or 6 months for the first year. A booster may be given in subsequent years at intervals of 1-12 months.

An alternate approach is to administer the initial immunization with a live recombinant carrier expressing the relevant antigen with the subsequent boost consisiting of liposomes carrying the relevant purified antigen given according to the schedules described above.

### EXAMPLE XII

Normal human subjects or subjects who are at high risk for the development of certain malignancies are vaccinated according to the schedule described above with subsequent boosters once every 6 months to 5 years. Examples of patients at high risk for the development of malignancy include those with familial polypoposis for colorectal cancer or those who smoke for lung cancer.

## Claims

1. An antitumor vaccine composition comprising, as active ingredient, a GA733-2 antigen, said antigen being encapsulated in, or covalently bound to, a liposome carrier.

2. The vaccine composition of claim 1 which further contains an additional synthetically prepared tumor-associated antigen.

3. An antitumor vaccine composition comprising, as active ingredient, an anti-idiotypic antibody that immunologically mimics the GA733-2 antigen encapsulated in, or covalently bound to, a liposome carrier.

4. Use of a GA733-2 antigen in the manufacture of a medicament comprising said GA733-2 antigen encapsulated in, or covalently bound to, liposome carrier, for vaccinating against a tumor that expresses the GA733-2 antigen.

5. The use of claim 4 wherein the medicament further contains an additional synthetically prepared tumor associated antigen.

6. The use of an anti-idiotypic antibody that immunologically mimics the GA733-2 antigen encapsulated in, or covalently bound to, a liposome carrier, in the manufacture of a medicament for vaccinating against a tumor that expresses the GA733-2 antigen.

## Patentansprüche

1. Antitumor-Impfstoffzusammensetzung, umfassend, als einen Wirkstoff, ein GA733-2-Antigen, welches Antigen eingekapselt ist in oder kovälent gebunden ist an einen Liposomenträger.

2. Impfstoffzusammensetzung nach Anspruch 1, welcher außerdem ein weiteres synthetisch hergestelltes Tumor-assoziiertes Antigen enthält.

3. Antitumor-Impfstoffzusammensetzung, umfassend, als einen Wirkstoff, einen anti-idiotypischen Antikörper, der immunologisch das in einen Liposomenträger eingekapselte oder kovalent daran gebundene GA733-2-Antigen nachahmt.

4. Anwendung eines GA733-2-Antigens in der Herstellung eines Medikaments, umfassend das in einen Liposomenträger eingekapselte oder kovalent daran gebundene GA733-2-Antigen, zum Impfen gegen einen Tumor, der das GA733-2-Antigen exprimiert.

5. Anwendung nach Anspruch 4, wobei das Medikament außerdem ein weiteres synthetisch hergestelltes Tumor-assoziiertes Antigen enthält.

6. Anwendung eines anti-idiotypischen Antikörpers, der immunologisch das in einen Liposom-Träger eingekapselte oder kovalent daran gebundene GA733-2-Antigen nachahmt, in der Herstellung eines Medikaments zum Impfen gegen einen Tumor, der das GA733-2-Antigen exprimiert.

## Revendications

1. Composition de vaccin antitumoral comprenant, en tant qu'ingrédient actif, un antigène GA733-2, ledit antigène étant encapsulé dans, ou lié de façon covalente à, un support sous forme de liposome.

2. Composition de vaccin selon la revendication 1 qui contient en outre un antigène supplémentaire préparé de façon synthétique associé à une tumeur.

3. Composition de vaccin antitumoral comprenant, en tant qu'ingrédient actif, un anticorps anti-idiotypique qui mime immunologiquement l'antigène GA733-2 encapsulé dans, ou lié de façon covalente, à un support sous forme de liposome.

4. Utilisation d'un antigène GA733-2 dans la fabrication d'un médicament comprenant ledit antigène GA733-2 encapsulé dans, ou lié de façon covalente à, un support sous forme de liposome, pour la vaccination contre une tumeur qui exprime l'antigène GA733-2.

5. Utilisation selon la revendication 4, dans laquelle le médicament contient en outre un antigène supplémentaire préparé de façon synthétique associé à une tumeur.

6. Utilisation d'un anticorps anti-idiotypique qui mime immunologiquement l'antigène GA733-2 encapsulé dans, ou lié de façon covalente à, un support sous forme de liposome, dans la fabrication d'un médicament pour la vaccination contre une tumeur qui exprime l'antigène GA733-2.
